(19) Europäisches Patentamt
European Patent Office
Office européen des brevets

(11) Numéro de publication: **0 207 039 B1**

# (12) FASCICULE DE BREVET EUROPEEN

(45) Date de publication du fascicule du brevet: **30.08.89**

(21) Numéro de dépôt: **86870068.3**

(22) Date de dépôt: **20.05.86**

(51) Int. Cl.⁴: **A23L 2/34**, A23L 3/34, A23C 9/12, A23F 5/14, A61K 7/28, A23G 3/00, C12N 9/00, C12H 1/00

(54) **Procédé pour éliminer l'oxygène dans les substances alimentaires et dentifrices et substances alimentaires et dentifrices contenant des enzymes à cet effet.**

(30) Priorité: **22.05.85 LU 85910**

(43) Date de publication de la demande: **30.12.86 Bulletin 86/52**

(45) Mention de la délivrance du brevet: **30.08.89 Bulletin 89/35**

(84) Etats contractants désignés: **AT BE CH DE FR GB IT LI LU NL SE**

(56) Documents cités:
**DE-A- 2 946 642**
**DE-A- 3 030 185**
**DE-A- 3 204 284**
**US-A- 3 920 521**

(73) Titulaire: **SYNFINA - OLEOFINA S.A., Parc Industrielle Zone D, B-6538 Manage(BE)**

(72) Inventeur: **Prieels, Jean-Paul, 7, avenue de Février, B-1200 Bruxelles(BE)**
Inventeur: **Maschelein, Charles, Foetstraets 72, B-1180 Bruxelles(BE)**
Inventeur: **Heilporn, Marc, 83, rue de l'Aqueduc, B-1050 Bruxelles(BE)**

(74) Mandataire: **Detrait, Jean-Claude, c/o LABOFINA S.A. Zone Industrielle C, B-6520 Feluy(BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

EP 0 207 039 B1

## Description

La présente invention se rapporte à un procédé pour éliminer l'oxygène présent dans les substances alimentaires et dentifrices. En particulier, la présente invention concerne un procédé pour éliminer aussi bien l'oxygène moléculaire que l'oxygène radicalaire présent dans les boissons et plus spécialement dans les boissons à pH acide comme la bière. La présente invention se rapporte également aux substances alimentaires et dentifrices comprenant une composition enzymatique, contenant de la superoxyde dismutase.

Dans le domaine de la conservation des aliments et des boissons en particulier, les effets néfastes de l'oxygène présent en solution aqueuse sont bien connus. En l'occurence, ces effets se traduisent par des changements de coloration et de changements de goût. Ces effets son dûs principalement auz radicaux libres formés lors de la réaction de l'oxygène avec des solutés réducteurs. Afin de réduire ces effets néfastes de l'oxygène, on utilise des agents anti-oxydants.

On connaît divers systèmes qui sont utilisés pour éliminer l'oxygène des aliments ou des boissons comme par exemple la catalase et les oxydo-réductases qui sont utilisés pour protéger les aliments contre les effets de dégradation dûs à l'oxygène; ce système permet notamment de réduire fortement la concentration en oxygène dissous, mais malheureusement il n'a aucun effet sur l'oxygène présent sous forme radicalaire, qui est responsable de mécanismes de dégradation.

On connaît également des systèmes faisant appel à la superoxyde dismutase seule, comme décrit notamment dans le brevet US 3 920 521. Cependant, la superoxyde dismutase ne consomme pas réellement l'oxygène et elle n'empêche pas toutes les réactions avec les différents réducteurs du milieu, puisque selon la réaction (1), elle capte l'ion superoxyde et reforme de l'oxygène.

$$(1)\quad O_2^{\cdot -} + O_2^{\cdot -} + 2\,H^+ \longrightarrow H_2O_2 + O_2$$

De plus, il est bien connu que la superoxyde dismutase n'a qu'un faible pouvoir anti-oxydant à pH inférieur à 5 et ceci est dû à l'existence éphémère du radical $O_2^{\cdot -}$ à pH acide comme le montrent les équations (2) et (3).

$$(2)\quad O_2^{\cdot -} + H^+ \rightleftharpoons HO_2^{\cdot} \quad (pK = 4{,}88)$$

$$(3)\quad H^+ + O_2^{\cdot -} \longrightarrow HO_2^{\cdot} + O_2 \quad (K = 2{,}5.10^8)$$

A l'heure actuelle, pour la conservation des aliments et en particulier des boissons, soit on quenche l'oxygène moléculaire, soit on ajoute un anti-oxydant qui aura pour effet de maintenir la teneur en oxygène à une certaine teneur que l'on s'impose.

Or il semble nécessaire pour améliorer considérablement le facteur conservation de capter à la fois l'oxygène moléculaire et l'oxygène radicalaire, de manière à écarter tout risque de dégradation oxydative au milieu.

La présente invention a pour but de remédier aux inconvénients mentionnés ci-dessus.

La présente invention a pour objet un procédé qui permette d'éliminer la présence d'oxygène tant sous forme moléculaire que sous forme radicalaire, dans les aliments, les boissons et en particulier dans les boissons dont le pH est inférieur à 5, et les dentifrices.

Le procédé de la présente invention pour éliminer l'oxygène tant moléculaire que radicalaire de substances alimentaires et dentifrices pouvant se dégrader par oxydation, est caractérisé en ce que l'on ajoute à ces substances une composition enzymatique comprenant une oxydase et son substrat, la catalase et la superoxyde dismutase.

La demanderesse a trouvé d'une manière inattendue qu'en ajoutant à des substances alimentaires et particulièrement à des boissons dont le pH est acide, une composition enzymatique comprenant à la fois une oxydase et son substrat, la catalase ainsi que la superoxyde dismutase, on obtient une durée de conservation de ces substances nettement améliorée concrétisée par une teneur en oxygène considérablement réduite dans le milieu.

Ceci est d'autant plus inattendu qu'il est bien connu que l'effet anti-oxydant de la superoxyde dismutase n'est que très éphémère à faible pH compte tenu de la constante de vitesse de la réaction (3) mentionnée ci-avant.

Parmi les substances qui peuvent être traitées, on peut notamment citer la mayonnaise, les pâtes de dentifrice, les chewing gums, la poudre de lait, les cafés solubles et autres analogues.

Le procédé de l'invention est particulièrement approprié pour traiter les boissons comme la bière dont le pH est voisin de 4, les jus de fruits ou de légumes et autres analogues.

La composition enzymatique utilisée dans le procédé de l'invention comprend nécessairement une oxydase et son substrat, sauf si ce dernier est déjà présent dans le milieu, la catalase, et la superoxyde dismutase. On peut dire que l'utilisation conjointe de ces trois enzymes donne lieu à un effet de synergie du point de vue des propriétés anti-oxydantes. En effet, lorsque ces enzymes sont utilisées seules ou même en mélange deux à deux, les résultats sont loin d'être probants, et n'incitent pas l'homme de l'art à les combiner.

La composition enzymatique utilisée dans le procédé de l'invention comprend tout d'abord une oxydase et son substrat. Comme oxydases appropriées, on peut utiliser la glucose oxydase, l'oxalate oxydase, la lactate oxydase, l'éthanol oxydase, l'ascorbate oxydase et des amino-acides oxydases; les substrats à utiliser sont évidemment le glucose, l'acide oxalique, l'acide lactique, l'éthanol, l'acide ascorbique et les aminoacides correspondants. Toutes ces oxydases sont disponibles commercialement, cependant pour des raisons de facilité, on préfère utiliser la glucose oxydase.

La quantité d'oxydase à mettre en œuvre est généralement comprise entre 0,1 et 1 ppm basé sur le milieu. Dans le cas où l'on utilise la glucose oxydase, on peut exprimer sa teneur en unités Sorett et celle-ci est généralement comprise entre 25 et 100 unités Sorett/l de milieu. L'unité Sorett est la quantité d'enzyme qui consomme 10 ml d'oxygène par minute à pH 5,6, en présence de 3% de glucose et d'air saturé en oxygène, à 30°C et en présence d'un excès de catalase. Les unités de concentration utilisées dans le présent texte sont exprimées en unités de poids par volume, par exemple mg/l pour ppm.

Le substrat est introduit dans le milieu, sauf s'il y est déjà présent en quantités suffisantes, à raison de 0,05 à 2% et de préférence de 0,1 à 1% basé sur le milieu.

La deuxième enzyme utilisée dans le procédé de l'invention est constituée par la catalase. La catalase est également une enzyme bien connue pour son action anti-oxydante lorsque utilisée avec une oxydase.

La catalase seule ou en mélange avec la superoxyde dismutase n'a pratiquement aucun effet antioxydant.

Selon un mode préféré du procédé de l'invention, on utilise la catalase à raison de 2.000 à 20.000 unités/litre de milieu et le plus souvent de 5.000 à 12.000 unités/litre de milieu. La catalase est disponible commercialement et dans certains cas, comme dans la glucose oxydase, elle y est déjà présente. Les unités de catalase sont des micromoles de produit formé ($O_2$) par ml, par minute et par mg de catalase.

La composition enzymatique comprend également de la superoxyde dismutase.

On introduit de la superoxyde dismutase dans le milieu à raison de 0,3 à 1,5 ppm et de préférence de 0,5 à 1 ppm. La superoxyde dismutase utilisée selon l'invention peut provenir de toute source appropriée comme l'érythrocyte ou encore de souches bactériennes marines comme Photobacterium phosphoreum, Phosphoreum leiognathi et Phosphobacterium sepia.

La demanderesse a trouvé d'une manière inattendue que l'utilisation conjointe de ces trois enzymes permettait une élimination de l'oxygène tant moléculaire que radicalaire de loin supérieure à ce que est obtenu avec les systèmes usuels. De plus, le procédé de l'invention est particulièrement applicable aux boissons à faible pH, notamment inférieur à 5, comme la bière.

On a d'ailleurs remarqué qu'avec le procédé de l'invention, on réduit la concentration en oxygène dissous dans la bière à quelques dizaines de ppb seulement, alors qu'avec les systèmes anti-oxydants usuels comme l'acide ascorbique, l'oxygène résiduel reste à un niveau compris entre 200 et 500 ppb.

Les exemples suivants servent à mieux illustrer l'invention, mais sans pour autant en limiter la portée.

Exemple 1

On a introduit dans une bouteille de bière de 25 cl de pH = 4,2 une compostion enzymatique comprenant de la glucose oxydase, le glucose comme substrat, la catalase et la superoxyde dismutase.

On a utilisé la glucose oxydase (GOD) à raison de 0,5 ppm par rapport au milieu. On a ensuite ajouté du glucose à raison de 0,1% basé sur le milieu ainsi que de la superoxyde dismutase (SOD) à raison de 1 ppm par rapport au milieu.

Après avoir introduit la composition enzymatique dans la bière, on a fermé la bouteille, et on a étudié la résistance du milieu au vieillissement, en portant la bière à une température de 50°C pendant 40 heures.

On a mesuré la résistance au vieillissement par le test à l'acide thiobarbiturique. Ce test permet de déterminer par une méthode colorimétrique la quantité de produits carbonylés formés par oxydation de la bière. Après refroidissement de la bière, on en prend un échantillon de 5 ml que l'on introduit dans un tube de 15 ml contenant 2 ml d'une solution contenant 0,33% d'acide thiobarbiturique dans un mélange 50/50 acide acétique-eau. Après mélange, on porte le tube à 60 °C pendant 1 heure, on refroidit et on lit l'absorbance à 530 nm. L'absorbance est indiquée en % de la valeur la plus élevée prise égale à 100%, une valeur élevée étant significative d'une meilleure résistance.

Avec la composition de l'invention, on a obtenu une valeur de 100%.

A titre de comparaison, on a déterminé la résistance d'une bière à laquelle on a ajouté uniquement la GOD, le glucose et la catalase, dans les mêmes proportions que ci-dessus. Comme contrôle, on a également testé une bière qui a été aérée. Les résultats furent les suivants:

- contrôle: 37,11% - GOD + glucose + catalase: 84,25%.

Ceci montre qu'en appliquant le procédé de l'invention, on obtient une bière ayant une beaucoup plus longue durée de vie.

Exemple 2

On a introduit dans une bouteille de bière de 25 cl de pH = 4,2 une composition enzymatique comprenant de la glucose oxydase à raison de 0,5 ppm basée sur le milieu, du glucose à raison de 0,1% basé sur le milieu et 0,5 ppm de SOD par rapport au milieu.

Après avoir introduit cette composition dans la bière, on a fermé la bouteille et on a étudié le pouvoir réducteur de la bière, en portant la bière à une température de 50°C pendant 40 heures.

On détermine le pouvoir réducteur de la bière en mesurant la réduction de complexe de dipyridyle/$Fe^{3+}$ (incolore) en dipyridyle/$Fe^{2+}$ (rouge) au moyen des substances réductrices de la bière.

On mesure l'absorbance (en %) à 510 nm.

La valeur de absorbance de la bière fraîche non aérée est prise comme égale à 100%.

Avec la composition de l'invention, on a obtenu une absorbance de 93,3%.

A titre de comparaison, on a déterminé le pouvoir réducteur d'une bière à laquelle on a ajouté uniquement la GOD, le glucose et la catalase dans les mêmes proportions que ci-dessus.

Pour contrôle, on a également pris un échantillon aéré. Les résultats furent les suivants:
- contrôle: 85,9% - GOD + glucose + catalase: 88,6%

Ceci montre que la bière traitée avec la composition de l'invention a un meilleur pouvoir réducteur.

Exemple 3

On a testé différents systèmes enzymatiques et autres à base d'acide ascorbique pour éliminer l'oxygène de la bière, responsable de perte de saveur et autres réactions de dégradation.

On a ainsi ajouté diverses compositions, reprises dans le tableau I, à différentes bouteilles de bière de 25 cl, bière dont le pH était de 4,2.

Après avoir injecté 10 ml d'air bouteille, on a ajouté la composition anti-oxydante, on a refermé la bouteille, on l'a agitée à 100 t/min à une température de 25°C pendant 64 heures.

On a déterminé la teneur en oxygène dissous dans la bière. Tous les résultats sont indiqués au tableau I.

**Tableau I**

| Essai | Composition | Teneur en $O_2$ dissous (ppm) |
|---|---|---|
| 1 | Contrôle – aéré | 3,19 |
| 2 | GOD (50 unités Sorett/l)<br>+ catalase ($10^4$ U)<br>+ glucose (à 0,25%) | 0,05 |
| 3 | Catalase ($10^4$ U) | 3,73 |
| 4 | SOD 0,5 ppm | 3,55 |
| 5 | SOD (0,5 ppm)<br>+ catalase ($10^4$ U) | 3,61 |
| 6 | GOD (50 unités Sorett)<br>+ catalase ($10^4$ U)<br>+ 0,5% glucose<br>+ SOD 0,5 ppm | 0,00 |
| 7 | Acide ascorbique (30 ppm) | 2,86 |

Cet exemple montre que seule la composition de l'invention montre une élimination complète de l'oxygène dissous.

## Revendications

1. Procédé pour empêcher l'auto-oxydation des substances alimentaires ou dentifrices pouvant se dégrader par oxydation avec l'oxygène moléculaire et/ou radicalaire, caractérisé en ce que l'on ajoute à ces substances une composition enzymatique comprenant une oxydase et son substrat, si ce dernier n'est déjà pas présent dans le milieu, la catalase et la superoxyde dismutase.

2. Procédé selon la revendication 1 selon lequel les substances sont choisies principalement parmi la mayonnaise, les pâtes de dentifrice, les chewing-gums, la poudre de lait, les cafés solubles, et les boissons comme la bière, les jus de fruits et les jus de légumes.

3. Procédé selon l'une quelconque des revendications 1 et 2 caractérisé en ce que l'on utilise une oxydase choisie dans le groupe comprenant la glucose oxydase, l'oxalate oxydase, la lactate oxydase, l'étha-

nol oxydase, l'ascorbate oxydase et les amino acides oxydases, et le substrat correspondant choisi parmi le glucose, l'acide oxalique, l'acide lactique, l'éthanol, l'acide ascorbique et les amino acides correspondants.

4. Procédé selon l'une quelconque des revendications 1 à 3 caractérisé en ce que l'on ajoute une oxydase à raison de 0,1 à 1 ppm basé sur le milieu, le substrat à raison de 0,05 à 2% basé sur le milieu, la catalase à raison de 2.000 à 20.000 unités/l de milieu et de préférence à raison de 5.000 à 12.000 unités, et la superoxyde dismutase à raison de 0,3 à 1,5 ppm et de préférence à raison de 0,5 à 1 ppm dans le milieu.

5. Procédé selon l'une quelconque des revendications précédentes caractérisé en ce que l'on ajoute à de la bière une composition enzymatique comprenant de 0,1 à 1 ppm de glucose oxydase par rapport à la bière, de 0,05 à 2% de glucose par rapport à la bière, de 2.000 à 20.000 unités de catalase par l de bière et de 0,3 à 1,5 ppm de superoxyde dismutase par rapport à la bière.

6. Substances alimentaires ou dentifrices pouvant se dégrader par oxydation, caractérisées en ce qu'elles contiennent une composition enzymatique pour en éliminer l'oxygène moléculaire et/ou radicalaire, correspondant à 0,1 à 1 ppm d'une oxydase par rapport au milieu, de 0,05 à 2% basé sur le milieu du substrat correspondant à l'oxydase, de 2.000 à 20.000 unités/l de milieu de catalase et de 0,3 à 1,5 ppm de superoxyde dismutase par rapport au milieu.

7. Substances selon la revendication 6, comprenant la mayonnaise, les pâtes de dentifrice, les chewing-gums, la poudre de lait, les cafés solubles ou les boissons comme la bière, les jus de fruits et de légumes.

8. Substances selon l'une quelconque des revendications 6 et 7 caractérisées en ce que l'oxydase est choisie parmi la glucose oxydase, l'oxalate oxydase, la lactate oxydase, l'éthanol oxydase, l'ascorbate oxydase et les aminoacides oxydases, et en ce que le substrat correspondant est choisi parmi le glucose, l'acide oxalique, l'acide lactique, l'éthanol, l'acide ascorbique et les amino acides correspondants.

9. Substances selon l'une quelconque des revendications 6 et 8 caractérisées en ce qu'elles comprennent de la bière comme substance alimentaire pouvant se dégrader par oxydation, et une composition enzymatique comprenant de 0,1 à 1 ppm de glucose oxydase par rapport à la bière, de 0,05 à 2% basé sur la bière de glucose comme substrat, de 2.000 à 20.000 unités/l de bière, de catalase et de 0,3 à 1,5 ppm de superoxyde dismutase par rapport à la bière.

**Patentansprüche**

1. Verfahren zur Verhinderung der Autoxidation von durch Oxidation mit molekularem und/oder radikalischem Sauerstoff abbaubaren Lebensmitteln und Zahnreinigungsmitteln, dadurch gekennzeichnet, daß man diesen Substanzen eine Enzym-Zubereitung zusetzt, die eine Oxidase und deren Substrat, wenn letzteres nicht schon im Medium vorhanden ist, sowie Katalase und Superoxid-Dismutase enthält.

2. Verfahren nach Anspruch 1, nach dem die Substanzen hauptsächlich aus Mayonnaise, Zahnpasten, Kaugummis, Milchpulver, löslichen Kaffees und Getränken wie Bier, Fruchsäften und Gemüsesäften gewählt werden.

3. Verfahren nach einem der Ansprüche 1 und 2, dadurch gekennzeichnet, daß eine Oxidase benutzt wird, die aus der Glukose-Oxidase, Oxalat-Oxidase, Laktat-Oxidase, Ethanol-Oxidase, Ascorbat-Oxidase und Aminosäure-Oxidasen umfassenden Gruppe gewählt wird, und das entsprechende Substrat, ausgewählt unter Glukose, Oxalsäure, Milchsäure, Ethanol, Ascorbinsäure und entsprechenden Aminosäuren.

4. Verfahren nach einem der Ansprüche 1 bis 3, dadurch gekennzeichnet, daß eine Oxidase in einer Menge von 0,1 bis 1 ppm auf der Basis des Mediums, das Substrat in einer Menge von 0,05 bis 2% auf der Basis des Mediums, die Katalase in einer Menge von 2.000 bis 20.000 Einheiten pro Liter Medium und vorzugsweise in einer Menge von 5.000 bis 12.000 Einheiten, und die Superoxid-Dismutase in einer Menge von 0,3 bis 1,5 ppm und vorzugsweise 0,5 bis 1 ppm dem Medium zugesetzt werden.

5. Verfahren nach einem der vorangegangenen Ansprüche, dadurch gekennzeichnet, daß dem Bier eine Enzym-Zubereitung zugesetzt wird, die 0,1 bis 1 ppm Glukose-Oxidase im Verhältnis zum Bier, 0,05 bis 2% Glukose im Verhältnis zum Bier, 2.000 bis 20.000 Einheiten Katalase pro Liter Bier und 0,3 bis 1,5 ppm Superoxid-Dismutase im Verhältnis zum Bier enthält.

6. Durch Oxidation abbaubare Lebensmittel oder Zahnreinigungsmittel, dadurch gekennzeichnet, daß sie zur Entfernung des molekularen und/oder radikalischen Sauerstoffs aus ihnen eine Enzymzubereitung enthalten, die 0,1 bis 1 ppm einer Oxidase im Verhältnis zum Medium, 0,05 bis 2% auf der Basis des Mediums des der Oxidase entsprechenden Substrats, 2.000 bis 20.000 Einheiten Katalase pro Liter Medium und 0,3 bis 1,5 ppm Superoxid-Dismutase im Verhältnis zum Medium entspricht.

7. Substanzen nach Anspruch 6, umfassend Mayonnaise, Zahnpasten, Kaugummis, Milchpulver, lösliche Kaffees oder Getränke, wie Bier, Frucht- und Gemüsesäfte.

8. Substanzen nach einem der Ansprüche 6 und 7, dadurch gekennzeichnet, daß die Oxidase aus Glukose-Oxidase, Oxalat-Oxidase, Laktat-Oxidase, Ethanol-Oxidase, Ascorbat-Oxidase und Aminosäure-Oxidasen gewählt wird, und daß das entsprechende Substrat aus Glukose, Oxalsäure, Milchsäure, Ethanol, Ascorbinsäure und entsprechenden Aminosäuren gewählt wird.

9. Substanzen nach einem der Ansprüche 6 bis 8, dadurch gekennzeichnet, daß sie Bier als durch Oxidation abbaubares Lebensmittel und eine Enzym-Zubereitung umfassen, die 0,1 bis 1 ppm Glukose-Oxidase im Verhältnis zum Bier, 0,05 bis 2% Glukose auf der Basis des Biers als Substrat, 2.000 bis 20.000 Einheiten Katalase pro Liter Bier und 0,3 bis 1,5 ppm Superoxid-Dismutase im Verhältnis zum Bier enthält.

**Claims**

1. Process for preventing the autoxidation of foodstuff materials or toothpastes capable of being degraded by oxidation with molecular and/or free-radical oxygen, characterized in that an enzyme composition comprising an oxidase and its substrate, if the latter is not already present in the medium, catalase and superoxide dismutase is added to these materials.

2. Process according to claim 1, according to which the materials are mainly chosen from mayonnaise, toothpastes, chewing gums, powdered milk, soluble coffees and drinks such as beer, fruit juices and vegetable juices.

3. Process according to either of claims 1 and 2, characterized in that an oxidase is used which is chosen from the group comprising glucose oxidase, oxalate oxidase, lactate oxidase, ethanol oxidase, ascorbate oxidase and amino-acid oxidases, and the corresponding substrate chosen from glucose, oxalic acid, lactic acid, ethanol, ascorbic acid and the corresponding amino acids.

4. Process according to any one of claims 1 to 3, characterized in that there are added an oxidase in the proportion of 0.1 to 1 ppm based on the medium, the substrate in the proportion of 0.05 to 2% based on the medium, catalase in the proportion of 2,000 to 20,000 units/l of medium and preferably in the proportion of 5,000 to 12,000 units, and superoxide dismutase in the proportion of 0.3 to 1.5 ppm and preferably in the proportion of 0.5 to 1 ppm based on the medium.

5. Process according to any one of the preceding claims, characterized in that there is added to the beer enzyme composition comprising from 0.1 to 1 ppm based on the beer of glucose oxidase, from 0.5 to 2% of glucose based on the beer, from 2,000 to 20,000 units of catalase per liter of beer and from 0.3 to 1.5 ppm of superoxide dismutase based on the beer.

6. Foodstuff materials or toothpastes capable of being degraded by oxidation, characterized in that they contain an enzyme composition for removing molecular and/or free-radical oxygen therefrom corresponding to 0.1 to 1 ppm based on the medium of an oxidase, from 0.05 to 2%, based on the medium of the substrate corresponding to the oxidase, from 2,000 to 20,000 units/l of medium of catalase and from 0.3 to 1.5 ppm based on the medium of superoxide dismutase.

7. Materials according to claim 6 comprising mayonnaise, toothpastes, chewing gums, powdered milk, soluble coffees or drinks such as beer, and fruit and vegetable juices.

8. Materials according to either one of claims 6 and 7, characterized in that the oxidase is chosen from glucose oxidase, oxalate oxidase, lactate oxidase, ethanol oxidase, ascorbate oxidase and amino-acid oxidases, and in that the corresponding substrate is chosen from glucose, oxalic acid, lactic acid, ethanol, ascorbic acid and the corresponding amino acids.

9. Materials according to any one of claims 6 to 8, characterized in that they comprise beer as a foodstuff material capable of being degraded by oxidation, and an enzyme composition comprising from 0.1 to 1 ppm based on beer of glucose oxidase, from 0.05 to 2% based on beer of glucose as substrate, from 2,000 to 20,000 units/l of beer of catalase and from 0.3 to 1.5 ppm based on beer of superoxide dismutase.